# EUROPEAN PATENT APPLICATION

(11) **EP 3 501 457 A1**
(43) Date of publication of application: **26.06.2019**
(21) Application number: 17306847.9
(22) Date of filing: 20.12.2017
(51) Int. Cl.: A61F 2/28

(54) **FLEXIBLE POROUS IMPLANT FIXATION SYSTEM**

(71) Applicant: Materialise N.V., 3001 Leuven (BE); OBL (Société Anonyme), 92320 Chatillon (FR)
(72) Inventor: ADAM, Jérémy, 92320 Châtillon (FR); GEEBELEN, Benjamin, 3001 Leuven (BE)
(74) Representative: Hirsch & Associés

(57) **Abstract**

The present disclosure relates to flexible porous implant fixation systems.

## Description

### BACKGROUND OF THE INVENTION

### Field of the Invention

The present disclosure relates to medical implants. More particularly, the present disclosure relates to flexible porous implant fixation systems.

### Description of the Related Technology

Porous or partly porous bone implants may be used in certain circumstances. They can be made porous for different reasons, such as to save weight, to decrease thermal conductivity, to achieve mechanical properties that come closer to those of the surrounding bone, and/or to allow bone ingrowth for better fixation.

When designing a bone implant to allow bone ingrowth for better fixation, implant designers face conflicting requirements: on the one hand the stiffness of the implant should be lower than that of the surrounding bone, and on the other hand the implant should be strong enough to survive accidental impacts.

In particular, implants that are stiffer than the surrounding bone can cause stress shielding. Stress shielding is the phenomenon in which the implant takes on most of the loading such that less of the loading is carried by the bone. This can lead to bone resorption. In other words, the opposite of bone ingrowth may occur. To lower the stiffness of a porous implant, one strategy is to lower the density of the porous structure.

However, a porous structure with a lower density often also has a reduced strength. This is detrimental, as it increases the risk of the implant breaking under accidental loads. To increase the strength of a porous implant, one strategy is to increase the density of its porous structure.

International patent application WO/2017/042366, which is incorporated by reference herein in its entirety, discloses a porous structure that allows reconciling both requirements: a low stiffness under normal loading and a high stiffness under accidental loading. In some embodiment, this porous structure comprises units that are connected by means of connection elements. Each pair of adjacent units has two surfaces that make contact upon larger-than-normal deformation. In this way, the connection elements can be dimensioned to deliver the low stiffness required to promote bone ingrowth when the surfaces do not touch, and the units can be designed to deliver, when their surfaces touch, the strength required to survive accidental loads.

Implants and porous structures that provide further novel and non-obvious improvements to the implants and porous structures described in WO/2017/042366, are further described herein.

### BRIEF DESCRIPTION OF THE DRAWINGS

FIG. 1 is an example of a porous structure having a body that is a helicoidal bar with integrated interlocking elements, according to certain aspects.
FIG. 2 is an example of a porous structure having a body that is a zig-zag shape with integrated interlocking elements, according to certain aspects.
FIG. 3 is an example of a porous structure having a body that is a perforated sheet with integrated interlocking elements, according to certain aspects.
FIG. 4 is an example of a porous structure having a body that is a perforated tube with integrated interlocking elements, according to certain aspects. In certain aspects, the body of FIG. 4 can be a skin for an internal porous structure.
FIG. 5A is an example of five bodies of units (e.g., porous unit bodies) of a porous structure, according to certain aspects.
FIG. 5B is an example of interlocking elements of a porous structure, according to certain aspects.
FIG. 5C is an example of connecting elements of a porous structure, according to certain aspects.
FIG. 5D is an example of a full porous structure formed from the elements of FIGs. 5A-5C, according to certain aspects.
FIG. 5E is an exploded view of an example of two units of a porous structure, each with a body and interlocking elements, according to certain aspects.
FIG. 6 is an exploded view of a hexagonal porous structure including two units and flexible connecting elements, according to certain aspects. In certain aspects, flexible connecting elements of the porous structure of FIG. 6 can make up a skin.
FIG. 7 is an example of two units of a porous structure connected by flexible connection elements, according to certain aspects.
FIG. 8 is an example of units of a porous structure stacked to form a linear structure and connected by flexible connection elements, according to certain aspects.
FIG. 9 is an example of porous units coupled to form a planar structure connected by flexible connection elements, according to certain aspects.
FIG. 10 is an example of porous units stacked and coupled to form a 3D structure connected by flexible connection elements, according to certain aspects.
FIG. 11 is an example of a screw with a helicoidal porous structure, according to certain aspects.
FIG. 12 is cut-away view of the example of a screw of FIG. 11. In certain aspects, the screw includes a screw head, screw tip, and outer shell, and a central core.
FIG. 13 is an example of a shaft of a screw with a porous structure formed as crossing helixes, according to certain aspects.
FIGs. 14A and 14B are examples of separate units of a porous structure for a shaft of a screw or bolt, according to certain aspects.
FIG. 14C is an example of separate units of FIGs. 14A and 14B coupled together, according to certain aspects.
FIG. 14D is an example of a helicoidal connecting element with screw thread for a shaft of a screw or bolt, according to certain aspects.
FIG. 14E is an example of a double-helix connecting element for a shaft of a screw or bolt, according to certain aspects.
FIG. 14F is an example of a shaft of a screw or bolt formed from the elements of FIGs. 14C-14E, according to certain aspects.
FIG. 15 is an example of a screw with two parallel helixes as a porous structure, according to certain aspects.
FIG. 16 is an example of a glenoid implant including porous structures, according to certain aspects.
FIG. 17 is an example of a glenoid implant (e.g., for large bone defects) including porous structures, according to certain aspects.
FIG. 18 is an example of a femur implant including porous structures, according to certain aspects.
FIG. 19 is an example of a knee implant including porous structures, according to certain aspects.
FIG. 20 is an example of a dental implant including porous structures, according to certain aspects.
FIG. 21A is an example of a body of an implant for an implant fixation system for bones (e.g, with a small cross section) including porous structures, according to certain aspects.
FIG. 21B is an example of a bolt of an implant for an implant fixation system for bones (e.g, with a small cross section) including porous structures, according to certain aspects.
FIG. 21C is an example of a plug of an implant for an implant fixation system for bones (e.g, with a small cross section) including porous structures, according to certain aspects.
FIG. 21D is an example an implant fixation system for bones (e.g, with a small cross section) including porous structures formed from the elements of FIGs. 21A-21C, according to certain aspects.
FIG. 22 is a schematic view of a prior art medical device.
FIG. 23 is an example of a system for designing and manufacturing 3D objects.
FIG. 24 illustrates a functional block diagram of one example of the computer shown in FIG. 23.
FIG. 25 shows a high-level process for manufacturing a 3D object using an additive manufacturing system.

### DETAILED DESCRIPTION OF INVENTION

The following description and the accompanying figures are directed to certain specific embodiments. The embodiments described in any particular context are not intended to limit this disclosure to the specified embodiment or to any particular usage. Those of skill in the art will recognize that the disclosed embodiments, aspects, and/or features are not limited to any particular embodiments. For example, reference to "a" layer, component, part, etc., may, in certain aspects, refer to "one or more."

Certain embodiments herein relate to implants that have reduced size as compared to some other porous structures having separate unit bodies, connecting elements, interlocking elements and skin.

For example, certain embodiments herein provide a porous structure design that further improves on the designs in WO/2017/042366. In particular, in certain embodiments, instead of structures comprising individual units that are connected by means of connection elements and have interlocking elements that only make contact upon large deformations, embodiments of porous structures described herein combine two or more of these elements to achieve a more compact design. One example embodiment includes one or more units that themselves provide the flexible behavior of the connection elements in WO/2017/042366, and that internally comprise two or more elements that restrict deformation and contribute to mechanical strength. In other words, the body of the unit at the same time functions as connection element and optionally even as skin. The restricting elements can be de-coupled elements that come into contact upon a certain level of deformation, such as opposing elements to restrict compression and interlocking elements to restrict extension. A combination of restriction of compression and extension can be obtained by designing interlocking elements that come into contact with each other upon excessive extension and that come into contact with the body of the unit upon excessive compression. Other embodiments are possible.

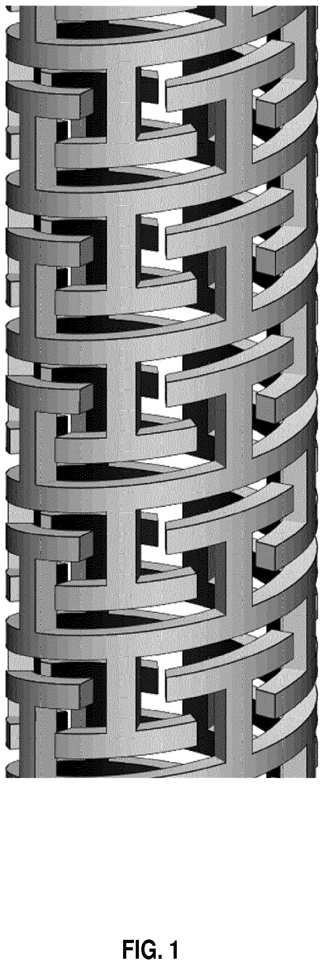

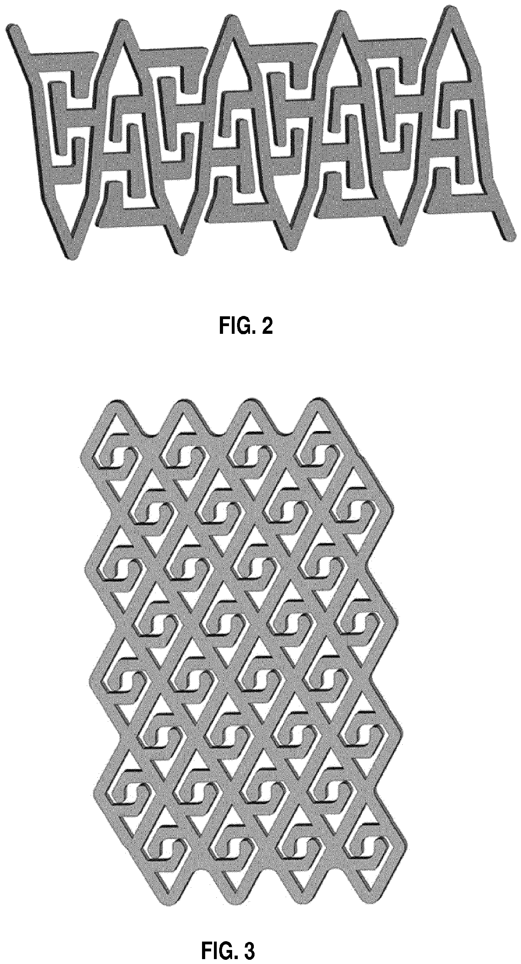

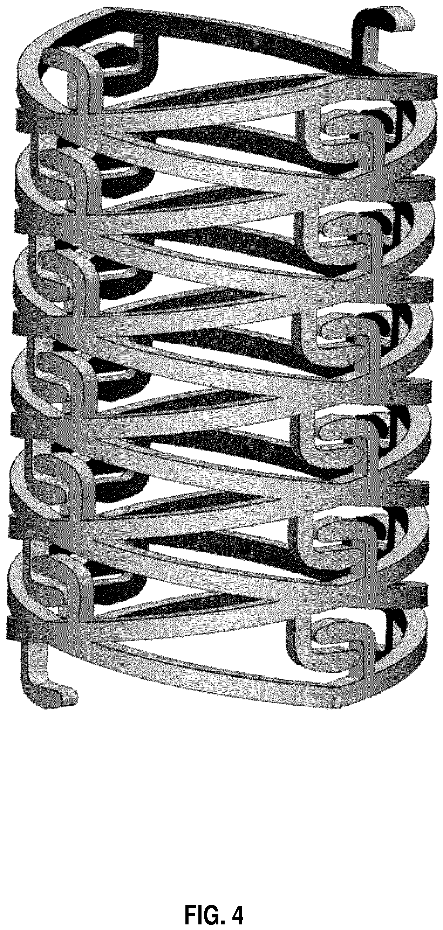

Examples embodiments of such units can have the shape of a helix (Figure 1), a zig-zag shape (Figure 2), a perforated structure (Figure 3, e.g. a honeycomb), a sheet (Figure 3) or a tube (Figure 4). Other shapes, such as 3-dimensional structures, layered structures, auxetic structures, prismatic structures, wavy designs, curly designs, staircase designs, stepped designs, corrugated designs, etc., are also possible embodiments.

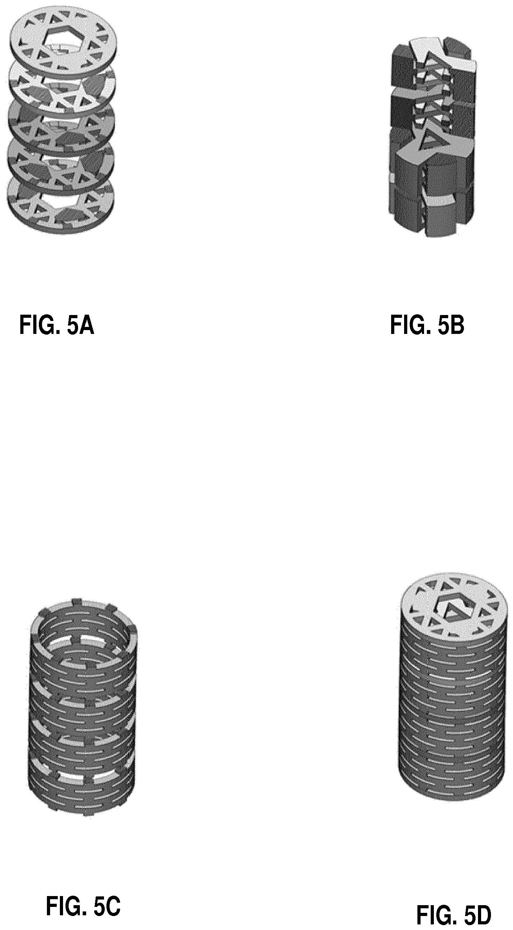

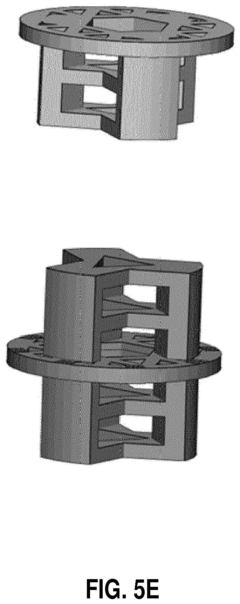

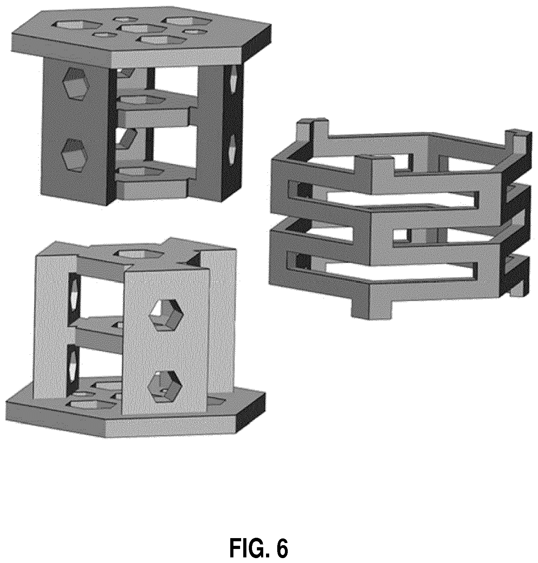

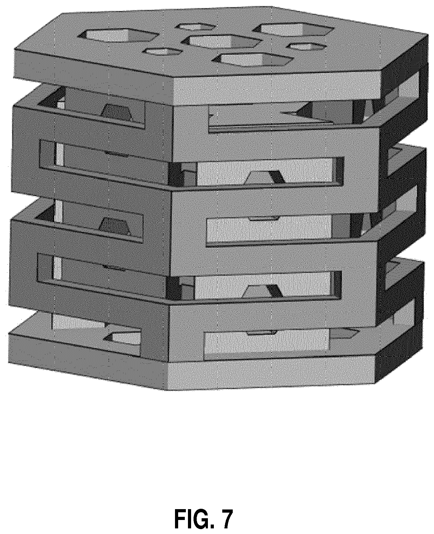

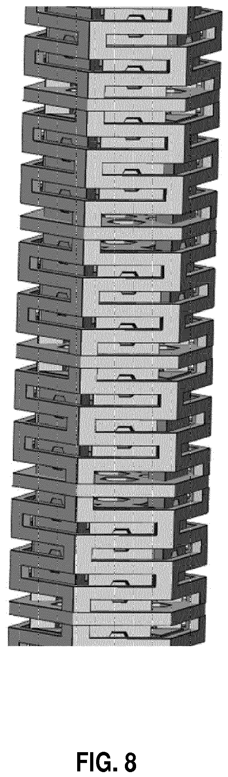

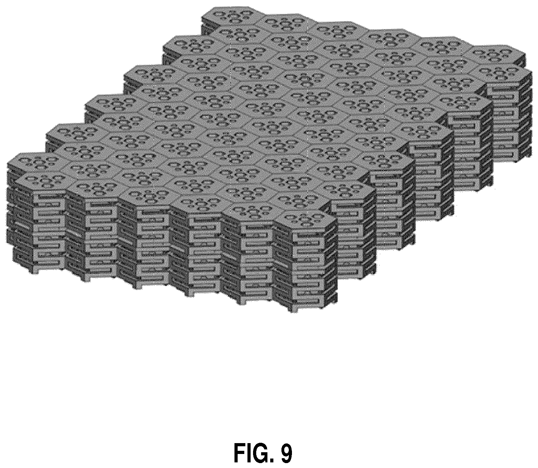

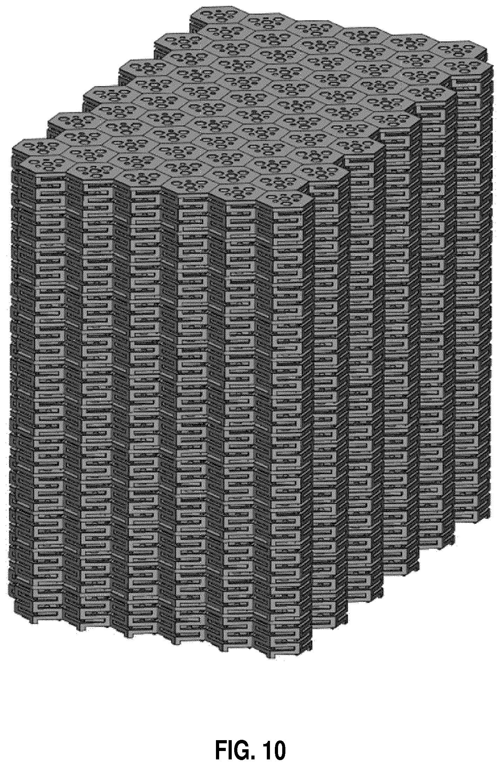

Another example embodiment combines the outer skin with the function of the connecting elements while maintaining identifiable unit bodies. Examples of such embodiments can be seen in Figures 5 to 10.

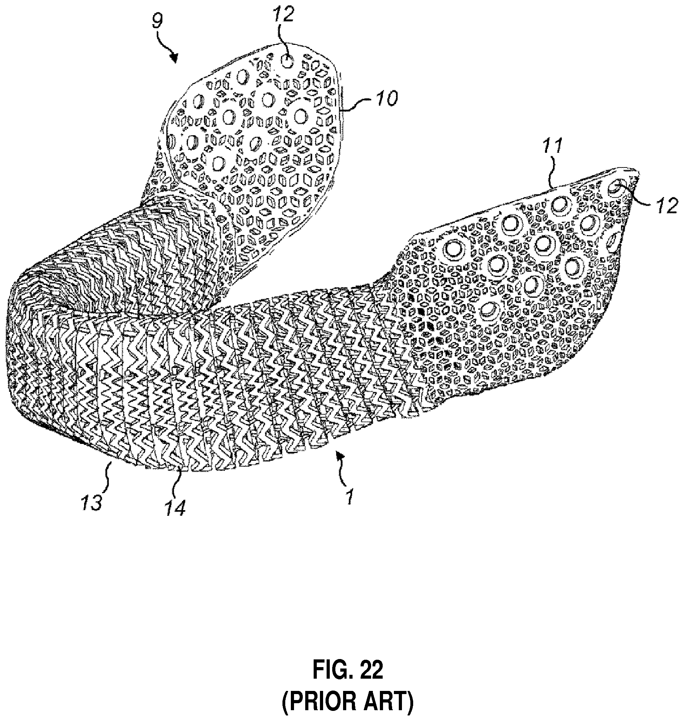

The prior art implant shown in FIG. 22 is connected to the bone by means of end portions 10 and 11. These end portions, though also porous, require so many reinforcements around the many fixation screw holes, that they may not achieve the low stiffness under normal loading that is targeted with the rest of the implant. This may be problematic, as the bone-implant interface-i.e. the osteotomy plane where the bone has been cut-is not loaded, and all the loads are redirected via a stiffer end portion and stiffer fixation screws towards the side of the bone. In other words, bone ingrowth is less promoted at the bone-implant interface than one would expect.

Certain embodiments herein provide a fixation system that has similar mechanical behavior as the porous structure of WO/2017/042366: a low stiffness under normal loads and a high strength under higher (i.e. accidental) loads. To accomplish this behavior, in certain embodiments, the fixation system uses an internal structure according to WO/2017/042366, an internal structure according to embodiments described herein, or a combination of both. In certain embodiments, the fixation system comprises one or more stems, pegs, screws and/or bolts, and optionally one or more plugs.

The stems, pegs, screws and/or bolts may have an internal structure according to the porous structure of WO/2017/042366 or according to the porous structure of embodiments described herein.

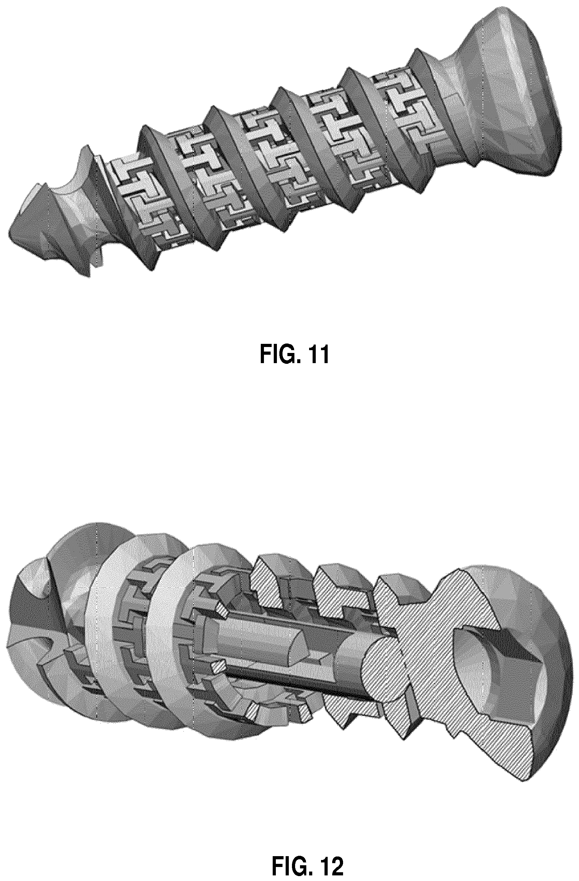

In some embodiments, the shafts of the stems, pegs, screws and/or bolts have an outer shell and an inner core. In certain embodiments, the outer shell can have a structure according to the helicoidal design of Figure 1. In the case of a screw or bolt, a screw thread can then follow the helix. An example of such a screw is shown in Figure 11-12. As shown, the outer shell comprises a helicoidal body with screw thread and interlocking elements. Because the outer shell is a helix, it offers little resistance to torsion until the interlocking elements touch. However, in some embodiments, torsion stiffness can be increased by having an inner core as shown in Figure 12, comprising longitudinally sliding elements, some connected to the screw head, some to the screw tip, which prevent torsion between head and tip.

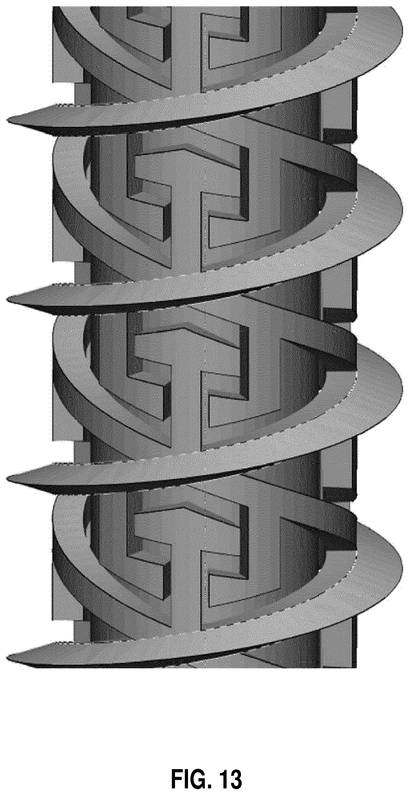

In certain embodiments, the shafts of the pegs, screws and/or bolts have an outer shell with a structure according to the crossing-helix design of Figure 4 (e.g., more than 2 helixes are possible, such as shown in Figure 13). A screw thread can then follow one helix. In this embodiment the presence of the crossing helix provides torsional stiffness. The central core can be left open, can be filled with the sliding elements of Figure 12, or can be filled in another way, e.g. with a porous structure. In certain embodiments, the central core does not prevent the compression and extension of the helicoidal body under normal loads. One example would be a porous structure comprising a multitude of mutually disconnected elements extending inwards from the outer shell.

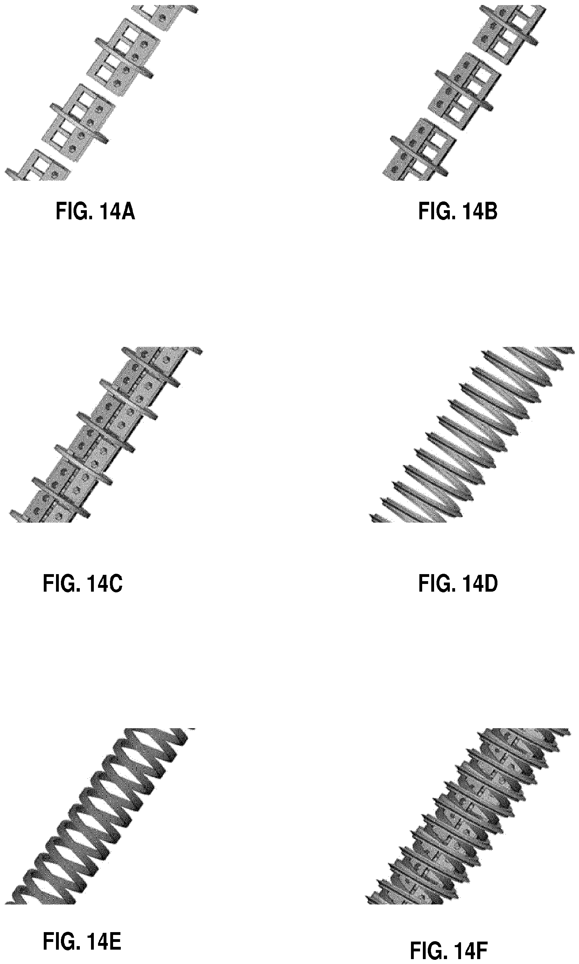

Another embodiment can have a single or crossing-helix body occupying the outer shell and interlocking elements occupying the inner core, as illustrated in Figure 14.

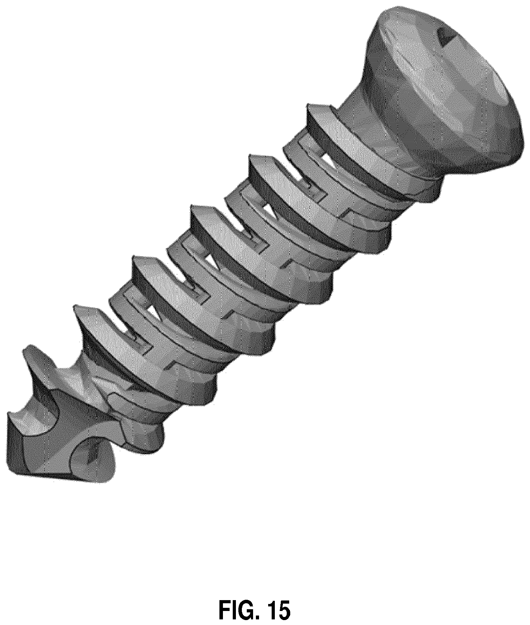

Another embodiment can have two parallel helixes connected at intervals as in Figure 15. This design restricts compression upon accidental loads, but not extension.

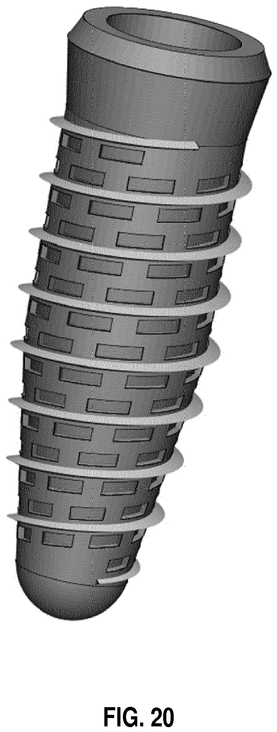

A particular example of a screw according to one embodiment is a dental implant as can be seen in Figure 20. The shaft of the dental implant can have any of the structures of the previous embodiments. In dental implants, infection due to bacteria present in the mouth is an alleged cause of bone loss. It may therefore be appropriate to provide the implant with an antibacterial coating and/or with a solid, smooth section of shaft where the implant protrudes from the bone.

For certain implants, loads between the implant and the bone are transmitted through shear stresses, which is not natural to bone. Bone is mainly capable of carrying compressive loads. Shear stresses localized around the fixation screws might lead to damage to the bone.

In some embodiments, the fixation system is used to attach an implant to an anatomy part of a patient (e.g. bone). To overcome the weaknesses mentioned, the fixation system may be designed in accordance with the prevailing loads to be expected at the implant/anatomy interface. Bone, for example, models itself to withstand prevailing stresses. An implant, in certain embodiments, may be designed to have an interface with the bone that is substantially perpendicular to the direction of these prevailing stresses so as to effectively transfer the loads from the bone to the implant. The fixation system in certain embodiments is also designed to maintain the transfer of the loads at the interface and not to divert the loads to another location or to convert the loads into other types of loads (e.g. from compression/tension to shear). Pegs, screws, and/or bolts may therefore be applied perpendicularly to the implant/anatomy interface.

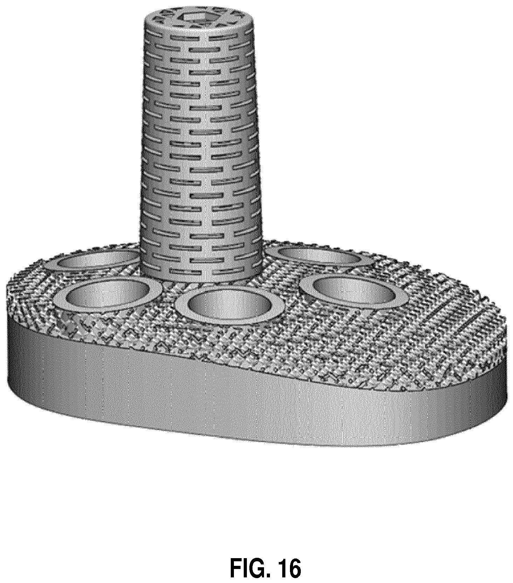

One embodiment of an implant fixation system is a peg or stem attached to the implant, such as can be seen in Figure 16 for a glenoid implant. In one embodiment, the implant has a solid metal core, perforations for screw insertion, a porous layer for bone ingrowth and a porous peg for stabilization and fixation. To avoid stress shielding and to promote bone ingrowth, one can use one of the porous structures described herein or of WO/2017/042366 for the peg, the porous layer (depicted here as a regular porous structure) and/or the fixation screws (not shown in the image).

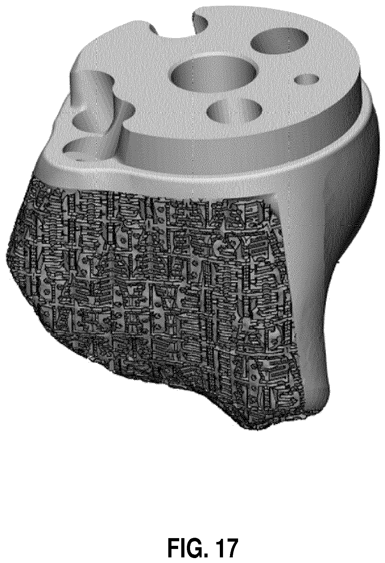

Another example embodiment of an implant fixation system is the combination of a glenoid implant such as can be seen in Figure 17 and one or more screws as seen in Figures 11-15. In one embodiment, the glenoid implant has a solid metal base, a porous structure according to other aspects of this invention and may be suited for large bone defects. The porous structure is oriented to accommodate the prevailing loads at the glenoid. At the side, the implant has a smooth wall to avoid muscles, tendons, nerves and blood vessels to be damaged or irritated as they move over the surface.

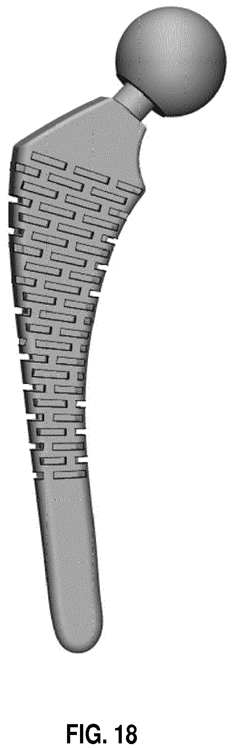

Another example embodiment is a femoral implant as can be seen in Figure 18. In one embodiment, the structure of Figure 5 is applied to part of the stem of the implant. In this way the stem can be designed to be flexible enough to avoid stress shielding and promote bone ingrowth, while still strong enough to withstand accidental loads.

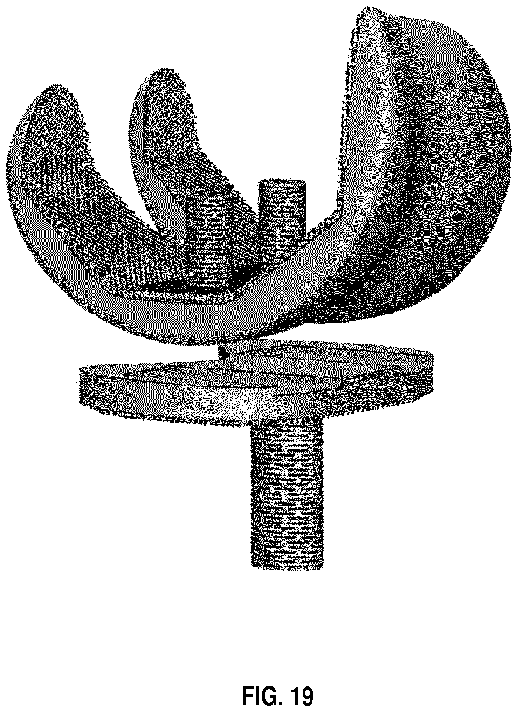

Another example embodiment is a knee implant as can be seen in Figure 19. In one embodiment, the knee implant, as shown, comprises a metal femoral component, a metal tibial component and a polymer spacer (not shown). Each metal component has a solid metal core, one or more bone-facing surfaces and one or more pegs. The pegs can be cylindrical, conical, fin-like or of any other suitable shape. To promote ingrowth, the bone-facing surfaces can be covered in a porous structure. The porous structure can be a regular structure, a structure according to WO/2017/042366 or a structure according to the embodiments discussed herein. The structure may be oriented so as to accommodate the prevailing loading directions. To promote bone ingrowth and prevent stress shielding, the pegs may be designed to comprise one of the porous structures of WO/2017/042366 or of the embodiments discussed herein.

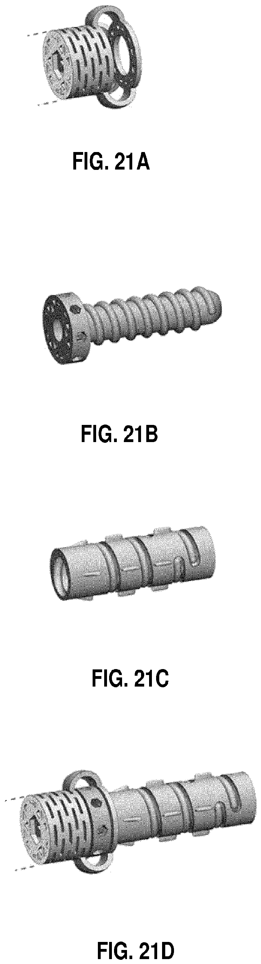

One embodiment, such as shown in FIG. 21D, provides a fixation system for elongate bones, such as a mandible or the diaphysis of a femur, tibia, fibula, humerus, radius, ulna, rib, metacarpal, metatarsal, phalange. Particularly in bones with a smaller cross section it can be a challenge to fixate a bone-replacing implant with a fixation system with an interface perpendicular to the axis of the bone and fixation screws substantially parallel to the axis of the bone. According to this embodiment, the implant fixation system comprises the following elements:
- a body as shown in FIG. 21A. This body replaces a section of the bone. In an embodiment, the body has a porous structure according to WO/2017/042366 or to embodiments disclosed in the present application. The body has an interface with the bone that is substantially perpendicular to the axis of the bone;
- a bolt or screw as shown in FIG. 21B. To overcome the challenge of the limited dimensions and still be able to align the bolt or screw within the bone/implant interface and substantially parallel to the axis of the bone, the head of the bolt or screw may lie within the volume of the body. The body may have an aperture extending further along the axis of the bolt or screw to allow the insertion of a screw driver. Alternatively, the head of the bolt or screw can be made accessible from the side, so that the bolt or screw can be fastened with a wrench or with a pin that grabs into a series of apertures in the head of the bolt or screw. In one embodiment, the bolt or screw has a porous structure as shown for the stems, pegs and screws above;
- a plug as shown in FIG. 21C. Particularly when there is no space to provide an aperture for a screw driver and the bolt or screw has to be fastened from the side, it's difficult for the surgeon to put sufficient pressure on the bolt or screw to drive it into the bone. In one embodiment, the implant fixation system therefore also comprises a plug. The hole for the plug can be pre-drilled, such as using a (patient-specific) drill guide. The plug can then be inserted into the hole, for example manually or using a hammer. The plug comprises external features to limit rotation (e.g. fins parallel to the axis of the plug). The plug also comprises at least one element to prevent extraction when the implant is fixed to the bone. This can be one or more elements that are pushed outward when the bolt is driven into the plug. Alternatively, it can be one or more anchoring screws or pins inserted at roughly 90° from the axis of the plug, such as are known from intramedullary nails. In one embodiment, the plug has a porous structure as shown for the stems, pegs and/or screws above.

One advantage of using a bolt and plug is that the plug can be designed to fit the shape of the bone. For example, the plug can be given a diameter best suitable for the cross section of the bone. For example, the plug can be dimensioned to fit between the cortical walls of the bone.

An important advantage of using an implant fixation system of which (e.g. all) elements have porous structures as described herein, is that not only the design of the implant, but also the fixation system promotes bone ingrowth.

In certain aspects, embodiments described herein, such as of implants, porous structures, flexible porous implant fixation systems, etc., may be manufactured using an additive manufacturing AM process.

AM processes are a material-addition approach to building parts, typically starting from a base material in liquid, solid sheet or powder form and consolidating the added material locally, in layer-by-layer fashion. Since the emergence of the first AM processes in the early 1990's, AM processes have been used as an alternative to conventional material-removal techniques such as milling, cutting or drilling or molding techniques, such as injection molding or extrusion molding, and have been shown to be especially effective in producing complex parts in a relatively short time, without dedicated tools such as molds or dies.

Among the best-known AM techniques are stereolithography (SLA), 3D-printing (3D-P), Selective Laser Sintering (SLS), Selective Heat Sintering (SHS), Selective Laser Melting (SLM), Direct Metal Laser Sintering (DMLS), Laser Beam Melting (LBM), and Electron Beam Melting (EBM). The techniques vary according to the tools used for consolidating the layers of a part, and according to materials that can be used in the techniques.

The systems and methods described herein may be performed using various additive manufacturing and/or three-dimensional (3D) printing systems and techniques. Typically, additive manufacturing techniques start from a digital representation (e.g., CAD file, such as STL, DWG, DXF, etc., mesh based model, voxel based model, etc.) of the 3D object to be formed. Generally, the digital representation is divided into a series of cross-sectional layers (e.g., perpendicularly to the Z-direction, meaning parallel to a build platform), or "slices," which are overlaid to form the object as a whole. The layers represent the 3D object, and may be generated using additive manufacturing modeling software executed by a computing device. For example, the software may include computer aided design and manufacturing (CAD/CAM) software. Information about the cross-sectional layers of the 3D object may be stored as cross-sectional data. An additive manufacturing (e.g., 3D printing) machine or system utilizes the cross-sectional data for the purpose of building the 3D object on a layer by layer basis. Accordingly, additive manufacturing allows for fabrication of 3D objects directly from computer generated data of the objects, such as computer aided design (CAD) files or STL files. Additive manufacturing provides the ability to quickly manufacture both simple and complex parts without tooling and without the need for assembly of different parts.

Additive manufacturing processes generally include providing energy from an energy source (e.g., a laser, an electron beam, etc.) to solidify (e.g., polymerize) layers of building material (e.g., plastic, metal, etc.). For example, the additive manufacturing machine may selectively apply energy from an energy source to (e.g., scan) the building material based on a job file. The job file may include information regarding slices of a digital representation of an object or objects to be built using an additive manufacturing process. For example, 3D objects represented by CAD files may be arranged in a virtual build volume corresponding to the build volume of an additive manufacturing device. Optionally, support structures may be added to the 3D objects in the virtual build volume (e.g., to improve build quality, heat dissipation, reduce deformation, etc.) The resulting 3D objects may be divided into layers or slices, as discussed. The job file, accordingly, may include slices (e.g., a stack of slices) of the 3D objects, and parameters of the additive manufacturing machine for building the 3D objects.

For example, for each slice, the job file may include information regarding a scanning pattern for the energy source to apply energy to (e.g., laser to scan, electron beam to scan, etc.) the physical layer of building material corresponding to that slice. It should be noted that as discussed herein, the terms slice and layer may be used interchangeably. The scanning pattern may include one or more vectors that each indicates a spatial position to apply the energy to the layer of building material and a direction to apply the energy to the building material (e.g., a direction to move the laser beam, electron beam, or other energy source over the building material while scanning).

An additive manufacturing machine builds an object on a layer by layer basis by applying energy to (e.g., scanning) the layers of building material according to the scanning pattern for each individual layer as indicated in a job file. For example, the additive manufacturing machine may scan a first layer of physical building material corresponding to a first slice of a digital representation of an object according to the scanning pattern for the first slice. The additive manufacturing machine may then scan a second layer of building material corresponding to a second slice adjacent to the first slice according to the scanning pattern for the second slice. The additive manufacturing machine continues scanning layers of building material corresponding to all the slices in the job file, until the layer corresponding to the last slice is scanned.

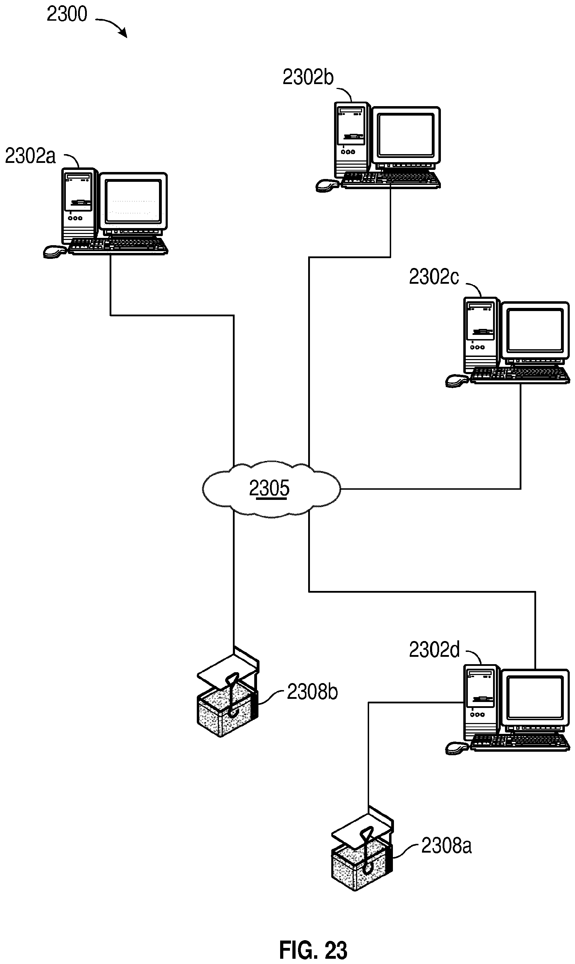

Embodiments of the invention may be practiced within a system for designing and manufacturing 3D objects. Turning to Figure 23, an example of a computer environment suitable for the implementation of 3D object design and manufacturing is shown. The environment includes a system 2300. The system 2300 includes one or more computers 2302a-2302d, which can be, for example, any workstation, server, or other computing device capable of processing information. In some aspects, each of the computers 2302a-2302d can be connected, by any suitable communications technology (e.g., an internet protocol), to a network 2305 (e.g., the Internet). Accordingly, the computers 2302a-2302d may transmit and receive information (e.g., software, digital representations of 3-D objects, commands or instructions to operate an additive manufacturing device, etc.) between each other via the network 2305.

The system 2300 further includes one or more additive manufacturing devices (e.g., 3-D printers) 23025a-23025b. As shown the additive manufacturing device 23025a is directly connected to a computer 2302d (and through computer 2302d connected to computers 2302a-2302c via the network 2305) and additive manufacturing device 23025b is connected to the computers 2302a-2302d via the network 2305. Accordingly, one of skill in the art will understand that an additive manufacturing device 23025 may be directly connected to a computer 2302, connected to a computer 2302 via a network 2305, and/or connected to a computer 2302 via another computer 2302 and the network 2305.

It should be noted that though the system 2300 is described with respect to a network and one or more computers, the techniques described herein also apply to a single computer 2302, which may be directly connected to an additive manufacturing device 23025. Any of the computers 2302a-2302d may be configured to function as the computing device described with respect to FIGs. 1-21. Further, any of the computers 2302a-2302d may be configured to perform the operations described herein.

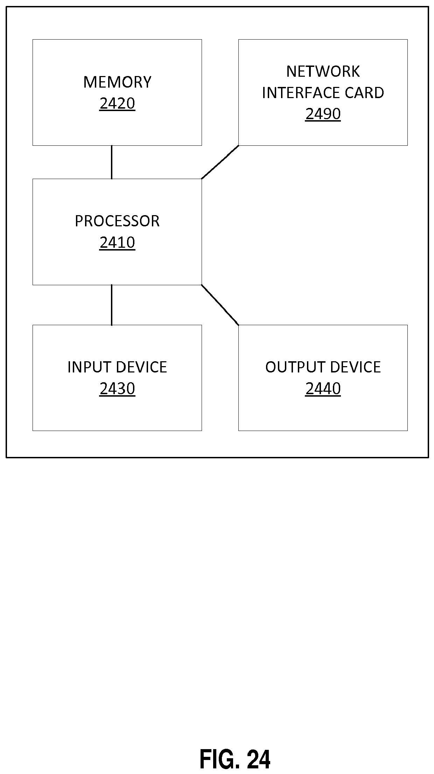

FIG. 24 illustrates a functional block diagram of one example of a computer of FIG. 23. The computer 2302a includes a processor 2410 in data communication with a memory 2420, an input device 2430, and an output device 2440. In some embodiments, the processor is further in data communication with an optional network interface card 2490. Although described separately, it is to be appreciated that functional blocks described with respect to the computer 2302a need not be separate structural elements. For example, the processor 2410 and memory 2420 may be embodied in a single chip.

The processor 2410 can be a general purpose processor, a digital signal processor (DSP), an application specific integrated circuit (ASIC), a field programmable gate array (FPGA) or other programmable logic device, discrete gate or transistor logic, discrete hardware components, or any suitable combination thereof designed to perform the functions described herein. A processor may also be implemented as a combination of computing devices, e.g., a combination of a DSP and a microprocessor, a plurality of microprocessors, one or more microprocessors in conjunction with a DSP core, or any other such configuration.

The processor 2410 can be coupled, via one or more buses, to read information from or write information to memory 2420. The processor may additionally, or in the alternative, contain memory, such as processor registers. The memory 2420 can include processor cache, including a multi-level hierarchical cache in which different levels have different capacities and access speeds. The memory 2420 can also include random access memory (RAM), other volatile storage devices, or non-volatile storage devices. The storage can include hard drives, optical discs, such as compact discs (CDs) or digital video discs (DVDs), flash memory, floppy discs, magnetic tape, and Zip drives.

The processor 2410 also may be coupled to an input device 2430 and an output device 2440 for, respectively, receiving input from and providing output to a user of the computer 2302a. Suitable input devices include, but are not limited to, a keyboard, buttons, keys, switches, a pointing device, a mouse, a joystick, a remote control, an infrared detector, a bar code reader, a scanner, a video camera (possibly coupled with video processing software to, e.g., detect hand gestures or facial gestures), a motion detector, or a microphone (possibly coupled to audio processing software to, e.g., detect voice commands). Suitable output devices include, but are not limited to, visual output devices, including displays and printers, audio output devices, including speakers, headphones, earphones, and alarms, additive manufacturing devices, and haptic output devices.

The processor 2410 further may be coupled to a network interface card 2490. The network interface card 2490 prepares data generated by the processor 2410 for transmission via a network according to one or more data transmission protocols. The network interface card 2490 also decodes data received via a network according to one or more data transmission protocols. The network interface card 2490 can include a transmitter, receiver, or both. In other embodiments, the transmitter and receiver can be two separate components. The network interface card 2490, can be embodied as a general purpose processor, a digital signal processor (DSP), an application specific integrated circuit (ASIC), a field programmable gate array (FPGA) or other programmable logic device, discrete gate or transistor logic, discrete hardware components, or any suitable combination thereof designed to perform the functions described herein.

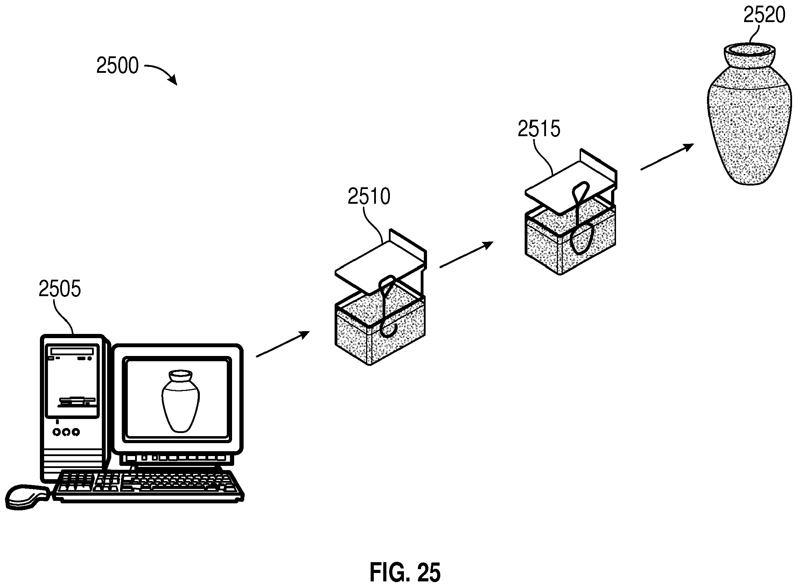

FIG. 25 illustrates a process 2500 for manufacturing a 3-D object or device. As shown, at a step 2505, a digital representation of the object is designed using a computer, such as the computer 2302a. For example, 2-D or 3-D data may be input to the computer 2302a for aiding in designing the digital representation of the 3-D object. Continuing at a step 2510, information is sent from the computer 2302a to an additive manufacturing device, such as additive manufacturing device 23025, and the device 23025 commences the manufacturing process in accordance with the received information. At a step 2515, the additive manufacturing device 25025 continues manufacturing the 3-D object using suitable materials, such as a liquid resin. At a step 2520, the object is finally built.

These suitable materials may include, but are not limited to a photopolymer resin, polyurethane, methyl methacrylate-acrylonitrile-butadiene-styrene copolymer, resorbable materials such as polymer-ceramic composites, metals, metal alloys, etc. Examples of commercially available materials are: DSM Somos® series of materials 7100, 8100, 9100, 9420, 10100, 11100, 12110, 14120 and 15100 from DSM Somos; ABSplus-P430, ABSi, ABS-ESD7, ABS-M30, ABS-M30i, PC-ABS, PC ISO, PC, ULTEM 9085, PPSF and PPSU materials from Stratasys; Accura Plastic, DuraForm, CastForm, Laserform and VisiJet line of materials from 3D-Systems; the PA line of materials, PrimeCast and PrimePart materials and Alumide and CarbonMide from EOS GmbH, Aluminum, CobaltChrome and Stainless Steel materials, MarangingSteel, Nickel Alloy, Titanium, and Titanium alloys. The VisiJet line of materials from 3-Systems may include Visijet Flex, Visijet Tough, Visijet Clear, Visijet HiTemp, Visijet e-stone, Visijet Black, Visijet Jewel, Visijet FTI, etc. Examples of other materials may include Objet materials, such as Objet Fullcure, Objet Veroclear, Objet Digital Materials, Objet Duruswhite, Objet Tangoblack, Objet Tangoplus, Objet Tangoblackplus, etc. Another example of materials may include materials from the Renshape 5000 and 7800 series. Further, at a step 2520, the 3-D object is generated.

Various embodiments disclosed herein provide for the use of computer software being executed on a computing device. A skilled artisan will readily appreciate that these embodiments may be implemented using numerous different types of computing devices, including both general-purpose and/or special-purpose computing system environments or configurations. Examples of well-known computing systems, environments, and/or configurations that may be suitable for use in connection with the embodiments set forth above may include, but are not limited to, personal computers, server computers, hand-held or laptop devices, multiprocessor systems, microprocessor-based systems, programmable consumer electronics, network PCs, minicomputers, mainframe computers, distributed computing environments that include any of the above systems or devices, and the like. These devices may include stored instructions, which, when executed by a microprocessor in the computing device, cause the computer device to perform specified actions to carry out the instructions. As used herein, instructions refer to computer-implemented steps for processing information in the system. Instructions can be implemented in software, firmware or hardware and include any type of programmed step undertaken by components of the system.

A microprocessor may be any conventional general purpose single- or multi-chip microprocessor such as a Pentium® processor, a Pentium® Pro processor, a 8051 processor, a MIPS® processor, a Power PC® processor, or an Alpha® processor. In addition, the microprocessor may be any conventional special purpose microprocessor such as a digital signal processor or a graphics processor. The microprocessor typically has conventional address lines, conventional data lines, and one or more conventional control lines.

Aspects and embodiments of the inventions disclosed herein may be implemented as a method, apparatus or article of manufacture using standard programming or engineering techniques to produce software, firmware, hardware, or any combination thereof. The term "article of manufacture" as used herein refers to code or logic implemented in hardware or non-transitory computer readable media such as optical storage devices, and volatile or non-volatile memory devices or transitory computer readable media such as signals, carrier waves, etc. Such hardware may include, but is not limited to, field programmable gate arrays (FPGAs), application-specific integrated circuits (ASICs), complex programmable logic devices (CPLDs), programmable logic arrays (PLAs), microprocessors, or other similar processing devices.

Various embodiments disclosed herein may be implemented using a computer or computer control system. A skilled artisan will readily appreciate that these embodiments may be implemented using numerous different types of computing devices, including both general-purpose and special-purpose computing-system environments or configurations. Examples of well-known computing systems, environments, and/or configurations that may be suitable for use in connection with the embodiments set forth above may include, but are not limited to, personal computers, server computers, hand-held or laptop devices, multiprocessor systems, microprocessor-based systems, programmable consumer electronics, network PCs, minicomputers, mainframe computers, distributed computing environments that include any of the above systems or devices, and the like. These devices may include stored instructions, which, when executed by a microprocessor in the computing device, cause the computer device to perform specified actions to carry out the instructions. As used herein, instructions refer to computer-implemented steps for processing information in the system. Instructions can be implemented in software, firmware or hardware and include any type of programmed step undertaken by components of the system.

A microprocessor may be any conventional general purpose single- or multi-chip microprocessor such as a Pentium® processor, a Pentium® Pro processor, a 8051 processor, a MIPS® processor, a Power PC® processor, or an Alpha® processor. In addition, the microprocessor may be any conventional special purpose microprocessor such as a digital signal processor or a graphics processor. The microprocessor typically has conventional address lines, conventional data lines, and one or more conventional control lines.

Aspects and embodiments of the inventions disclosed herein may be implemented as a method, apparatus or article of manufacture using standard programming or engineering techniques to produce software, firmware, hardware, or any combination thereof. The term "article of manufacture" as used herein refers to code or logic implemented in hardware or nontransitory computer readable media such as optical storage devices, and volatile or non-volatile memory devices or transitory computer readable media such as signals, carrier waves, etc. Such hardware may include, but is not limited to, field programmable gate arrays (FPGAs), application-specific integrated circuits (ASICs), complex programmable logic devices (CPLDs), programmable logic arrays (PLAs), microprocessors, or other similar processing devices.

## Claims

1. A flexible porous implant fixation system comprising:
a plurality of porous body units with integrated interlocking elements; and
one or more flexible connecting elements that connect the plurality of porous body units.
